# EUROPEAN PATENT SPECIFICATION

(11) **EP 4 232 828 B1**
(45) Date of publication and mention of the grant of the patent: **09.10.2024**
(21) Application number: 22725531.2
(22) Date of filing: 20.04.2022
(51) Int. Cl.: G01N 33/68

(54) **METHODS AND KITS FOR DIAGNOSING COVID-19 DISEASE**
VERFAHREN UND KITS ZUR DIAGNOSE DER COVID-19-KRANKHEIT
PROCÉDÉS ET KITS DE DIAGNOSTIC DE LA COVID-19

(30) Priority: 20.04.2021 GB 202105644
(43) Date of publication of application: 30.08.2023
(73) Proprietor: Stellenbosch University, 7600 Stellenbosch (ZA)
(72) Inventor: PRETORIUS, Etheresia, 7600 Stellenbosch (ZA)
(74) Representative: Forrest, Stuart
(86) International application number: PCT/IB2022/053669
(87) International publication number: WO 2022/224148

(56) References cited:
- MAIER CHERYL L ET AL: "COVID-19 patient plasma demonstrates resistance to tPA-induced fibrinolysis as measured by thromboelastography", JOURNAL OF THROMBOSIS AND THROMBOLYSIS, SPRINGER US, NEW YORK, vol. 52, no. 3, 7 April 2021 (2021-04-07), pages 766 - 771, XP037609630, ISSN: 0929-5305, [retrieved on 20210407], DOI: 10.1007/S11239-021-02438-Y
- PRETORIUS ETHERESIA ET AL: "Prevalence of amyloid blood clots in COVID-19 plasma", MEDRXIV, 29 July 2020 (2020-07-29), XP055943923, Retrieved from the Internet <URL:https://doi.org/10.1101/2020.07.28.20163543.> [retrieved on 20220719], DOI: 10.1101/2020.07.28.20163543
- PRETORIUS ETHERESIA ET AL: "Prevalence of readily detected amyloid blood clots in 'unclotted' Type 2 Diabetes Mellitus and COVID-19 plasma: a preliminary report", CARDIOVASCULAR DIABETOLOGY, vol. 19, no. 1, 1 December 2020 (2020-12-01), pages 193, XP055943731, Retrieved from the Internet <URL:https://cardiab.biomedcentral.com/track/pdf/10.1186/s12933-020-01165-7.pdf> DOI: 10.1186/s12933-020-01165-7
- ALBAB NEMAT D ET AL: "Inhibition of amyloid fibrillation, enzymatic degradation and cytotoxicity of insulin at carboxyl tailored gold-aryl nanoparticles surface", COLLOIDS AND SURFACES A : PHYSIOCHEMICAL AND ENGINEERINGS ASPECTS, ELSEVIER, AMSTERDAM, NL, vol. 586, 26 November 2019 (2019-11-26), XP085970829, ISSN: 0927-7757, [retrieved on 20191126], DOI: 10.1016/J.COLSURFA.2019.124279
- DOYKOV IVAN ET AL: "'The long tail of Covid-19' - The detection of a prolonged inflammatory response after a SARS-CoV-2 infection in asymptomatic and mildly affected patients [version 1; peer review: 2 approved]", F1000 RESEARCH, 19 November 2020 (2020-11-19), England, pages 1349, XP055944456, Retrieved from the Internet <URL:https://www.ncbi.nlm.nih.gov/pmc/articles/PMC7745182/pdf/f1000research-9-32778.pdf> [retrieved on 20220720], DOI: 10.12688/f1000research.27287.1
- MERTZ LESLIE: "Researchers Seek Answers for Millions With Long COVID-19", IEEE PULSE, IEEE, USA, vol. 12, no. 2, 16 April 2021 (2021-04-16), pages 17 - 21, XP011850071, ISSN: 2154-2287, [retrieved on 20210416], DOI: 10.1109/MPULS.2021.3066718
- PRETORIUS ETHERESIA ET AL: "Persistent Clotting Protein Pathology in Long COVID/ Post-Acute Sequelae of COVID-19 (PASC) is Accompanied by Increased Levels of Antiplasmin", RESEARCH SQUARE, 23 June 2021 (2021-06-23), XP055943693, Retrieved from the Internet <URL:https://assets.researchsquare.com/files/rs-649504/v1/b488dcf1-1b81-4f44-ae0f-51071eff3093.pdf?c=1631885374> [retrieved on 20220718], DOI: 10.21203/rs.3.rs-649504/v1
- KELL DOUGLAS B. ET AL: "A central role for amyloid fibrin microclots in long COVID/PASC: origins and therapeutic implications", BIOCHEMICAL JOURNAL, vol. 479, no. 4, 25 February 2022 (2022-02-25), GB, pages 537 - 559, XP055944121, ISSN: 0264-6021, Retrieved from the Internet <URL:https://watermark.silverchair.com/bcj-2022-0016c.pdf?token=AQECAHi208BE49Ooan9kkhW_Ercy7Dm3ZL_9Cf3qfKAc485ysgAAA90wggPZBgkqhkiG9w0BBwagggPKMIIDxgIBADCCA78GCSqGSIb3DQEHATAeBglghkgBZQMEAS4wEQQMrUmZu1Zps_untZCNAgEQgIIDkLOFY16owU74Fm1f8doHuUoiZQKOUKHCSitn0Y_L41Pfw3KU6CbLFazd0yshIKM6STbhOa9gtmsgumUk9ysAY> DOI: 10.1042/BCJ20220016

## Description

### BACKGROUND OF THE INVENTION

This invention relates to a method of diagnosing COVID-19 disease or post-acute COVID-19 syndrome in a subject using a detection method, including a fluorescent or microscopy detection method or any other method to detect for a presence of persistent anomalous (amyloid) clotlets in the sample, wherein the presence of persistent anomalous (or amyloid) clotlets in the sample is indicative of either acute COVID-19 disease or post-acute COVID-19 syndrome in the subject. The invention also relates to diagnostic kits for diagnosing acute COVID-19 disease or post-acute COVID-19 syndrome in a subject, comprising fluorescent dyes or fluorescent probes for detecting the presence of persistent clotlets in the sample, wherein the presence of persistent clotlets in the sample is indicative of COVID-19 disease post-acute COVID-19 syndrome in the subject.

Acute COVID-19 is a contagious disease, caused by severe acute respiratory syndrome coronavirus 2 (SARS-CoV-2). Symptoms of COVID-19 are varied, including fever, cough, fatigue, breathing difficulties, and loss of smell and taste. SARS-CoV-2 spreads mainly through person-to-person contact and infections range from mild to deadly. In addition to various respiratory symptoms, COVID-19 has also been shown to cause various coagulopathies.

COVID-19 has had a devastating effect on our society. More so on patients with various cardiovascular co-morbidities, including cancer. Commonalities between patients with COVID-19 and cardiovascular disease include increased circulating inflammatory biomarkers and significant blood clotting abnormalities. In inflammatory conditions, anomalous clotting is known to occur. During acute COVID-19 infection, severed pathological clotting is present. COVID-19 patients and those with severe cardiovascular disease may also suffer from thrombocytopenia, which may be life-threatening.

Aside from acute COVID-19 infection, patients may also suffer from long-term health issues related to COVID-19, recently termed as LongCOVID or post-acute COVID-19 syndrome. LongCOVID presents as a collection of lingering symptoms for 90 days or more after patients have recovered, that may persist in an estimated 25-35%, regardless of infection severity; even after the patient no longer tests positive for the presence of SARS-CoV-2 virus.

To date, testing for acute COVID-19 has mainly focussed on detecting the presence of SARS-CoV-2 virus, or of antibodies produced in response to infection by SARS-CoV-2 virus. Nucleic acid tests target different parts of the SARS-CoV-2 viral genome to amplify and thus detect the presence of viral RNA. Antigen tests have also been developed to detect viral proteins present in a sample using antibodies that bind these proteins. Antibodies are usually detectable approximately 14 days after the onset of viral infection and form the basis of antibody testing, which provides an indication of a prior SARS-CoV-2 infection. However, none of these testing methods is useful for diagnosing LongCOVID. Further, the standard method of detecting the presence and severity of clotting pathologies in COVID-19 patients is based on CT chest scans for pneumonia, which have high sensitivity but low specificity, and are neither cheap nor universally available. In addition, Thromboelastography (TEG), a point of care technique is also used to determine the extent of clotting disfunction in acute COVID-19. Pathology laboratories also determine levels of D-dimer and fibrinogen concentrations to relate these concentrations to severity of clotting disfunction during acute COVID-19.

The inventors of the present invention have developed a method for diagnosing COVID-19, and in particular LongCOVID or post-acute COVID-19 syndrome, based on abnormal clotting, abnormal fibrinolysis and pathological and anomalous clotlets which may persist in patients who have suffered from acute COVID-19, but who have clinically recovered, and are not infectious any more.

Pretorius, E., Venter, C., Laubscher, G.J., Lourens, P.J., Steenkamp, J. and Kell, Douglas B (2020) Prevalence of amyloid blood clots in COVID-19 plasma, medRxiv. doi:10.1101/2020.07.28.20163543 discloses a fluorescent microscopy analysis of platelet poor plasma (PPP) samples to which no thrombin has been added from patients with COVID-19. According to the disclosure, microclots can be detected in the native plasma of COVID-19 patient, and in particular that such clots are amyloid in nature as judged by a standard fluorogenic stain.

Maier CL, Sarker T, Szlam F, Sniecinski RM (2021) COVID-19 patient plasma demonstrates resistance to tPA-induced fibrinolysis as measured by thromboelastography, J Thromb Thrombolysis. doi: 10.1007/s11239-021-02438-y discloses that clots from COVID-19 patients contains a higher fibrin content compared to standard controls and show resistance to fibrinolysis induced by tPA. Maier CL *et al* also discloses that fibrinolytic clinical efficacy of thrombolytics may be reduced in COVID-19 patients.

### SUMMARY OF THE INVENTION

The present invention relates to a method of diagnosing post-acute COVID-19 syndrome in a subject using a fluorescent or microscopy detection method to detect for a presence of persistent anomalous (amyloid) clotlets in the blood (or plasma) sample, wherein the presence of such clotlets in the blood (or plasma) sample is indicative of post-acute COVID-19 syndrome in the subject. Disclosed but not forming part of the invention is a diagnostic kit for diagnosing COVID-19 disease or post-acute COVID-19 syndrome in a subject comprising a fluorescent dye or fluorescent probe for detecting a presence of amyloid clotlets in the sample, wherein the presence of persistent anomalous (amyloid) clotlets in the sample is indicative of COVID-19 disease post-acute COVID-19 syndrome in the subject. The methods according to the present invention are particularly suitable for point-of-care diagnosis of COVID-19 disease, particularly post-acute COVID-19 syndrome.

According to the present invention there is provided for a method of diagnosing post-acute COVID-19 syndrome in a subject comprising:
(i) providing a sample, preferably a blood sample, obtained from the subject; and
(ii) treating the sample with a fluorescent dye or fluorescent probe;
(iii) using a fluorescent detection or microscopy method to detect the fluorescent dye or fluorescent probe thereby detecting for a presence of persistent anomalous amyloid clotlets having a diameter of 1 to 1000 µm in the sample,
wherein the presence of persistent anomalous (amyloid) clotlets in the sample is indicative of post-acute COVID-19 syndrome in the subject. In a first embodiment of the method of diagnosing post-acute COVID-19 syndrome in a subject, the method further comprises a step of preparing platelet poor plasma (PPP) from the blood sample.

According to a second embodiment of the method of diagnosing post-acute COVID-19 syndrome, the method further comprises treating the sample with a fibrinolytic enzyme prior to the step of using the fluorescent or microscopy detection method to detect for the presence of persistent anomalous (amyloid) clotlets in the sample.

In a further embodiment of the method of diagnosing post-acute COVID-19 syndrome, the fibrinolytic enzyme is selected from the group consisting of trypsin, protein kinases, plasmin, anistreplase, desmoteplase, streptokinase, urokinase, nattokinase, lumbrokinase, serrapeptase, papain, DNase, bromelain, and honokiol.

According to the invention,the method of diagnosing post-acute COVID-19 syndrome comprises treating the sample with a fluorescent dye or fluorescent probe and detecting the fluorescent dye or fluorescent probe. It will be appreciated by those of skill in the art that numerous fluorescent dyes or probes are known in the art. Preferably the fluorescent dye or fluorescent probe is a marker dye or probe that binds to protein, in particular anomalous protein, and/or plasma clotting proteins. A non-limiting example of such a protein is Thioflavin T (ThT). The fluorescent dye or fluorescent probe may further be a labelled antibody that binds to a plasma clotting protein.

According to another embodiment of the method of diagnosing post-acute COVID-19 syndrome, the fluorescent or microscopy detection method is fluorescence microscopy, confocal microscopy, fluorescence spectrofluorometry or flow cytometry.

### Disclosures not forming part of the invention

Disclosed but not forming part of the invention is a method of diagnosing COVID-19 disease in a subject comprising:
(i) providing a sample, preferably a blood sample, obtained from the subject;
(ii) optionally treating the sample with a fibrinolytic enzyme; and
(iii) using a fluorescent or microscopy detection method to detect for a presence of persistent anomalous (amyloid) clotlets in the sample,
wherein the presence of persistent anomalous (amyloid) clotlets in the sample is indicative of acute COVID-19 disease or post-acute COVID-19 syndrome in the subject.

According to the disclosed method of diagnosing COVID-19 disease in a subject, the COVID-19 disease may be acute COVID-19 disease, but is preferably post-acute COVID-19 syndrome.

According to the disclosed method of diagnosing COVID-19 disease, the method may further comprise a step of preparing platelet poor plasma (PPP) from the blood sample.

According to the disclosed method of diagnosing COVID-19 disease, the fibrinolytic enzyme may be an enzyme selected from the group consisting of trypsin, protein kinases, plasmin, anistreplase, desmoteplase, streptokinase, urokinase, nattokinase, lumbrokinase, serrapeptase, papain, DNase, bromelain, and honokiol.

According to the disclosed method of diagnosing COVID-19 disease, the fluorescent detection method may comprise treating the sample with a fluorescent dye or fluorescent probe and detecting the fluorescent dye or fluorescent probe. It will be appreciated by those of skill in the art that numerous fluorescent dyes or probes are known in the art. Preferably the fluorescent dye or fluorescent probe is a marker dye or probe that binds to protein, in particular anomalous protein, and/or plasma clotting proteins. A non-limiting example of such a protein is Thioflavin T (ThT). The fluorescent dye or fluorescent probe may further be a labelled antibody that binds to a plasma clotting protein.

According to the disclosed method of diagnosing COVID-19 disease, the fluorescent or microscopy detection method may be fluorescence microscopy, confocal microscopy, fluorescence spectrofluorometry, or flow cytometry.

Disclosed but not forming part of the invention is a diagnostic kit for diagnosing post-acute COVID-19 syndrome in a subject comprising:
(i) a fluorescent dye or fluorescent probe for detecting a presence of persistent anomalous (amyloid) clotlets in a sample obtained from the subject;
wherein the presence of persistent anomalous (amyloid) clotlets in the sample is indicative of post-acute COVID-19 syndrome in the subject.

According to the disclosed diagnostic kit for diagnosing post-acute COVID-19 syndrome, the sample may be a blood sample. Preferably, the sample is a platelet poor plasma (PPP) sample.

According the disclosed diagnostic kit for diagnosing post-acute COVID-19 syndrome, the diagnostic kit may further comprise a fibrinolytic enzyme for treating the sample.

According to the disclosed diagnostic kit for diagnosing post-acute COVID-19 syndrome, the fibrinolytic enzyme may be selected from the group consisting of, trypsin, protein kinases, plasmin, anistreplase, desmoteplase, streptokinase, urokinase, nattokinase, lumbrokinase, serrapeptase, papain, DNase, bromelain, and honokiol.

According to the disclosed the diagnostic kit for diagnosing post-acute COVID-19 syndrome, the fluorescent dye or fluorescent probe may be a fluorescent dye or fluorescent probe that is detected using fluorescence microscopy, confocal microscopy, fluorescence spectrofluorometry, or flow cytometry. It will be appreciated by those of skill in the art that numerous fluorescent dyes or probes are known in the art. Preferably the fluorescent dye or fluorescent probe is a marker dye or probe that binds to protein, in particular anomalous protein, and/or plasma clotting proteins. A non-limiting example of such a protein is Thioflavin T (ThT). The fluorescent dye or fluorescent probe may further be a labelled antibody that binds to a plasma clotting protein.

Disclosed but not forming part of the invention is a diagnostic kit for diagnosing COVID-19 disease in a subject comprising:
(i) a fibrinolytic enzyme for treating a sample obtained from the subject; and
(ii) a fluorescent dye or fluorescent probe for detecting a presence of persistent anomalous (amyloid) clotlets in the sample;
wherein the presence of persistent anomalous (amyloid) clotlets in the sample is indicative of COVID-19 disease in the subject.

According to the disclosed diagnostic kit for diagnosing COVID-19 disease in a subject, the COVID-19 disease may be acute COVID-19 disease or post-acute COVID-19 syndrome.

According to the disclosed diagnostic kit for diagnosing COVID-19 disease, the sample may be a blood sample. Preferably, the sample is a platelet poor plasma (PPP) sample.

According to the disclosed diagnostic kit for diagnosing COVID-1 9 disease, the fibrinolytic enzyme may be selected from the group consisting of, trypsin, protein kinases, plasmin, anistreplase, desmoteplase, streptokinase, urokinase, nattokinase, lumbrokinase, serrapeptase, papain, DNase, bromelain, and honokiol.

According to the disclosed diagnostic kit for diagnosing COVID-1 9 disease, the fluorescent dye or fluorescent probe may be a fluorescent dye or fluorescent probe that is detected using fluorescence microscopy, confocal microscopy, fluorescence spectrofluorometry, or flow cytometry. It will be appreciated by those of skill in the art that numerous fluorescent dyes or probes are known in the art. Preferably the fluorescent dye or fluorescent probe is a marker dye or probe that binds to protein, in particular anomalous protein, and/or plasma clotting proteins. A non-limiting example of such a protein is Thioflavin T (ThT). The fluorescent dye or fluorescent probe may further be a labelled antibody that binds to a plasma clotting protein.

Disclosed but not forming part of the invention is a diagnostic kit as defined herein for use in a method of diagnosing COVID-19 disease in a subject. In particular, the diagnostic kit may be for use in a method of diagnosing post-acute COVID-19 syndrome in a subject.

Disclosed but not forming part of the invention is the use of the diagnostic kit as defined herein in a method of diagnosing COVID-19 disease in a subject, particularly in a method of diagnosing post-acute COVID-19 syndrome in a subject.

Disclosed but not forming part of the invention is a method of treating a subject diagnosed with post-acute COVID-19 syndrome according to the methods of the invention, by administering to the subject an anti-coagulant and/or an anti-platelet medication to the subject. It will be appreciated by those of skill in the art that several anti-coagulants and anti-platelet medications are known. For example, the anti-coagulant may be selected from the group consisting of warfarin, rivaroxaban, dabigatran, apixaban, edoxaban, and heparin. In a non-limiting embodiment, therapeutic (not prophylactic) doses of either fondaparinux, unfractionated heparin or Low Molecular Weight (LMW) heparin may be used as the anti-coagulant medication.

The anti-platelet medication may be selected from the group consisting of aspirin, clopidogrel, cangrelor, prasugrel, ticagrelor, ticlopidine. Further, the anti-platelet medication may be a glycoprotein (GP) IIβ/IIIα inhibitor, such as abciximab, eptifibatide, and/or tirofiban.

According to the disclosed method of treating the subject may include administering to the subject dual antiplatelet therapy (DAPT). In particular, the DAPT may include administration of one or more anti-platelet medications together with an anti-coagulant selected from the group consisting of: LMW heparin, fondaparinux, and direct oral anticoagulants (DOACs). More particularly, the DAPT may be determined on an individualized basis for each subject.

### BRIEF DESCRIPTION OF THE FIGURES

Non-limiting embodiments of the invention will now be described by way of example only and with reference to the following figures:
**Figure 1****:** Representative micrographs of plasma smears from: (A) healthy patients; (B) diabetes patients; and (C) acute COVID-19 patients, after fibrinolytic digestion.
**Figure 2****:** Representative micrographs of plasma smears, after fibrinolysis treatment with trypsin fibrinolysis from four patients who suffered from acute COVID-19, but who recovered. They are now suffering from LongCOVID two months after their recovery from acute COVID-19.
**Figure 3****:** Representative micrographs of plasma smears from: (A) a healthy patient before COVID-19 infection; (B) the same patient 2 months after acute COVID-19 suffering from LongCOVID symptoms; and C) a second example of an individual suffering from LongCOVID, 2 months after COVID-19.

### DETAILED DESCRIPTION OF THE INVENTION

The present invention will now be described more fully hereinafter with reference to the accompanying drawings, in which some, but not all embodiments of the invention are shown.

The invention as described should not be limited to the specific embodiments disclosed and modifications and other embodiments are intended to be included within the scope of the invention. Although specific terms are employed herein, they are used in a generic and descriptive sense only and not for purposes of limitation.

As used throughout this specification and in the claims which follow, the singular forms "a", "an" and "the" include the plural form, unless the context clearly indicates otherwise.

The terminology and phraseology used herein is for the purpose of description and should not be regarded as limiting. The use of the terms "comprising", "containing", "having" and "including" and variations thereof used herein, are meant to encompass the items listed thereafter and equivalents thereof as well as additional items.

The present disclosure relates to a method of diagnosing COVID-19, including acute COVID-19, and LongCOVID or post-acute COVID-19, based on the presence of anomalous blood clotlets, particularly anomalous blood clotlets that are resistant to fibrinolysis in a sample. The present invention relates to a method of diagnosing post-acute Covid-19 syndrome. The disclsoure also relates to a kit, which is not claimed, for diagnosing post-acute COVID-19 comprising a fluorophore that binds to persistent anomalous (amyloid) clotlets (with origin plasma clotting proteins) before or after treatment with a fibrinolytic enzyme or to a kit for diagnosing acute COVID-19 comprising a fibrinolytic enzyme and a fluorophore that binds to persistent anomalous (amyloid) clotlets (with origin plasma clotting proteins).

The inventors have shown that abnormal clotting, abnormal fibrinolysis and pathological clotlets may persist in patients who have suffered from acute COVID-19, but who have clinically recovered, and are not infectious any more. Such abnormal blood clotting in patients suffering from both COVID-19 and LongCOVID may be resistant to fibrinolysis. The presence of these persistent hypofibrinolytic clotlets may be used as diagnostic tool for diagnosis of acute COVID-19, as well as LongCOVID.

The inventors have developed a method of diagnosing COVID-19, including acute COVID-19, and LongCOVID or post-acute COVID-19, based on the presence of anomalous blood clotlets, particularly anomalous blood clotlets that are resistant to fibrinolysis in a sample. This method could repeatedly and successfully distinguish between healthy plasma samples, type 2 diabetes and acute COVID-19 and LongCOVID.

The inventors of the present invention have found that both acute COVID-19 and LongCOVID plasma samples contain large persistent anomalous clotted plasma that are of an amyloid nature. The presence of these clotlets are known to cause severe clotting pathologies in acute COVID-19. Further, these clotlets are present in blood plasma both before and after fibrinolytic digestion in LongCOVID, but not after fibrinolytic digestion in healthy individuals or those with inflammatory diseases like type 2 diabetes. Further, no induction of clotting by the addition of a coagulation factor, such as thrombin, is required in the method. The presence of these persistent anomalous (amyloid) clotlets may be used to diagnose LongCOVID, which may further be confirmed with fibrinolysis - as the persistent anomalous (amyloid) clotlets are resistant to fibrinolysis. Further, the presence of fibrinolytic persistent anomalous (amyloid) clotlets may be used as the basis of diagnosing acute COVID-19.

Further, the finding that these persistent anomalous (amyloid) clotlets are present in LongCOVID patients has led the inventors to investigate treatment of patients diagnosed with LongCOVID with known anticoagulants, with a positive effect on the symptoms of these patients.

As used herein, the term "COVID-19 disease" refers to a contagious disease caused by severe acute respiratory syndrome coronavirus 2 (SARS-CoV-2) and includes both "acute COVID-19 disease" and "post-acute COVID-19 syndrome", also referred to as "LongCOVID".

As used herein, the term "acute COVID-19 disease" refers to COVID-19 disease which is characterised by one or more symptoms selected from: fever, dry cough, tiredness, aches and pains, sore throat, diarrhoea, conjunctivitis, headache, loss of taste or smell, a rash on skin, discolouration of fingers or toes, difficulty breathing or shortness of breath, chest pain or pressure, and loss of speech or movement, and which typically lasts for 5 days up to 4 weeks.

As used herein, the terms "post-acute COVID-19 syndrome" and "LongCOVID" refer to a COVID-19 disease characterised by persistent symptoms and/or delayed or long-term complications of SARS-CoV-2 infection beyond 4 weeks from the onset of symptoms. Post-acute COVID-9 syndrome may further be divided into two categories, namely: (1) subacute or ongoing symptomatic COVID-19, which includes symptoms and abnormalities present from 4-12 weeks beyond acute COVID-19; and (2) chronic or post-COVID-19 syndrome, which includes symptoms and abnormalities persisting or present beyond 12 weeks of the onset of acute COVID-19 and not attributable to alternative diagnoses.

As used herein, the term "persistent anomalous (amyloid) clotlets" refers to an aggregation of plasma clotting proteins that have an amyloid nature that have a diameter of between 1 to 1000 µm in diameter. Amyloids are aggregates of proteins characterised by a fibrillar morphology of 7-13 nm in diameter, a β-sheet secondary structure and the ability to be stained by particular dyes that bind the plasma clotting proteins. Typically such anomalous clotlets are a collection of plasma clotting proteins that mainly comprise of the clotting protein fibrin(ogen), but may also contain various of the other known clotting proteins (e.g. prothrombin, thrombin, Factor XIII) as well as various proteins and microparticles that might have an origin from endothelial cells, platelets, erythrocytes or white blood cells.

As used herein, the term "sample" refers to a sample obtained from a biological source, a "biological sample", including a blood, blood plasma, lymph, tissue or urine sample. In a non-limiting embodiment, said sample is a blood plasma sample.

The term "detecting" as used herein includes qualitative and/or quantitative detection or measuring levels of the persistent anomalous (amyloid) clotlets, with or without reference to a control. Typically, the anomalous (amyloid) clotlets may be detected by a "fluorescent detection method" or by visualizing the clotlets with a light microscope. Suitable fluorescent detection methods may include fluorescence microscopy, confocal microscopy, fluorescence spectrofluorometry or flow cytometry of the sample. Typically, the detection comprises contacting the sample with selective reagents such as probes, dyes or other ligands, and thereby detecting the presence, or measuring the level or amount, of protein of interest present in the sample. The contacting may be made under any condition suitable for a detectable complex, formed between the reagent and the nucleic acids or proteins of the sample. In one embodiment, the sample may be treated with a dye or probe, in particular a fluorescent dye or probe, that can be detected by one or more of the abovementioned fluorescent detection methods when it is bound to anomalous (amyloid) clotlets. In another embodiment, binding of the fluorescent dye or probe to anomalous (amyloid) clotlets may be visualised with the naked eye.

If fluorescence microscopy is used for the detection of persistent anomalous (amyloid) clotlets, the % of anomalous (amyloid) clotting can be calculated using the thresholding method. This method allows a measurement of area of amyloid signal. The RGB images are opened in imaged or any similar program, each image is calibrated by setting the scale (calculated using the image pixel size and the known size of the scale bar). Each image is then converted to black and white (8 bit, this is adjusted under the image type setting). Thereafter the background intensity is adjusted to white (255) and the black amyloid signal is thresholded. The Huang setting may be used during thresholding. Huang's method is an optimization method which finds the optimal threshold value by minimizing the measures of fuzziness. The black anomalous (amyloid) area is analyzed using the analyze particle setting (wherein the particle size that is measured is from 1 to infinity). The particle size setting allows for exclusion of any background signal that might not be true amyloid signal. Statistical analysis may be done using Graphpad Prism or any similar statistical program known in the art.

In one embodiment of the present invention, the methods of the invention may include a step of treating the sample with a fibrinolytic enzyme, and the diagnostic kits of the invention may include a fibrinolytic enzyme for treating a sample.

As used herein, the term "fibrinolytic enzyme" refers to any enzyme that is capable of stimulating the dissolution of a blood clot and the term "fibrinolysis" refers to the enzymatic breakdown of a blood clot by the action of such fibrinolytic enzyme. Examples of such fibrinolytic enzymes include, trypsin, protein kinases, plasmin, anistreplase, desmoteplase, streptokinase, urokinase, nattokinase, lumbrokinase, serrapeptase, papain, DNase, bromelain, and honokiol. According to a non-limiting embodiment, the fibrinolytic enzyme is preferably trypsin or protein kinases.

The methods of the invention may further comprise a step of comparing biomarker levels with a control reference or with a reference value.

As used herein, the term "control" relates to a healthy subject, or a subject who does not suffer from COVID-19 disease or post-acute COVID-19 syndrome. The control may also be a subject with a known or baseline level of anomalous (amyloid) clotlets. The control may also be a subject with no detectable persistent anomalous (amyloid) clotlets, after treatment with a fibrinolytic enzyme. Preferably, said control is a healthy subject that does not suffer from systemic long-term inflammation. Typically, amyloid clotlets range in control samples from a healthy subject are less than 1 µm and in control samples from subject with an inflammatory condition range from 1 to 5 µm.

Typically, a "reference value" is determined experimentally, empirically, or theoretically. Preferably, the reference value is a known or baseline level of anomalous (amyloid) clotlets in a healthy individual or a population of healthy individuals, wherein the term "healthy individual" denotes a subject which is known to be healthy, i.e. which does not suffer, and never has suffered, from COVID-19 disease or post-acute COVID-19 syndrome. Alternatively, the reference value may be in relation to the level of the anomalous (amyloid) clotlets in a known disease state and is the mean level of the anomalous (amyloid) clotlets in a diseased individual or a population of diseased individuals, wherein the term "diseased individual" denotes a subject which is known to suffer, or have suffered, from COVID-19 disease or post-acute COVID-19 syndrome, or alternatively from another disease that results in anomalous (amyloid) clotlets in blood plasma.

In some instances, the kits according to the disclosure may be provided together with instructions for use.

The following examples are offered by way of illustration and not by way of limitation.

### EXAMPLE 1

### Fibrinolysis and fluorescent microscopy of plasma

### Patient Samples

10 acute COVID-19 samples, 6 LongCOVID samples, 5 Type 2 diabetes samples and 5 healthy age-matched samples were analysed.

LongCOVID patients were diagnosed with LongCOVID symptoms at least 2 months after a negative PRC test confirmed their recovery from acute COVID-19.

Both COVID-19 and LongCOVID patients (when they were initially diagnosed with acute COVID-19) were confirmed to be COVID-positive by PRC testing at a pathology laboratory.

Type 2 Diabetes patients were confirmed by clinician to have suffered from diabetes form at least 6 months prior to blood sample collection.

Healthy individuals were confirmed by pathology results of blood biomarkers to not suffer from any long-term chronic systemic inflammation.

### Fibrinolysis

Plasma samples were diluted tenfold in 10 mM ammonium bicarbonate buffer and the protein concentration was determined at A280 nm using a nanodrop (Thermo) spectrophotometer. The samples were adjusted to the lowest sample concentration (5mg/mL) and equal volumes of each sample was removed for digestion (40uL). To each sample a total of 4 micrograms of trypsin in 10 µL was added for a final volume of 50 µL and at an enzyme to substrate ratio of 1:50. Plasma protein digestion was allowed to proceed at 37 degrees Celsius for 18 hours. After 18 hours, the samples were removed from the heating block and centrifuged at 14 000 x g for 30 min at ambient temperature to pellet the digestion resistant portion. The supernatant was then removed and 10µL sample was left at the bottom of the tubes.

Plasma samples of healthy controls and diabetes individuals were fully digested using the above digestion methods. The digested plasma was a clear fluid with no visible deposits. In contrast, all of the COVID-19 and LongCOVID samples contained insoluble deposits at the bottom of the tube after trypsin digestion.

### Fluorescent microscopy

Following digestion, 0.5 µL Thioflavin T (ThT), at a 5 miliMolar final concentration, was added to the 10 µL plasma and left for 30 minutes. Thereafter, 3 µL of the sample was placed onto a microscope slide, left for 30 seconds, and a coverslip was placed onto the sample. The samples were viewed using a Zeiss Axio Observer 7 fluorescent microscope with a Plan-Apochromat 63x/1.4 Oil DIC M27 objective (Carl Zeiss Microscopy, Munich, Germany). The excitation wavelength for ThT was set at 450nm to 488nm and the emission at 499nm to 529nm.

The % of anomalous (amyloid) clotting can be calculated using the thresholding method. This method was not performed in the present analysis due to sample size and because the fluorescent signal was so significant in the micrographs that such calculations were deemed unnecessary.

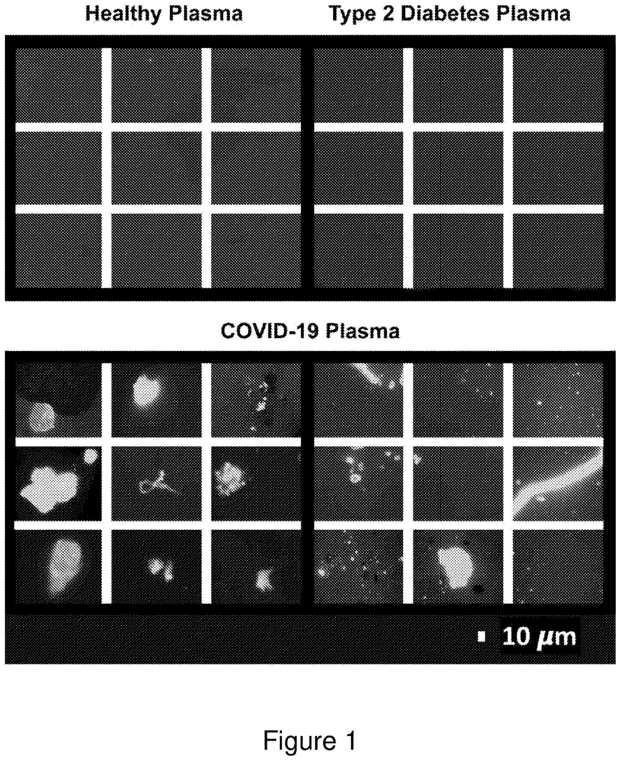

**Figures 1** **A to C** show representative micrographs of plasma smears from healthy individuals, diabetes patients and COVID-19 patients, after fibrinolysis treatment with trypsin fibrinolysis, respectively.

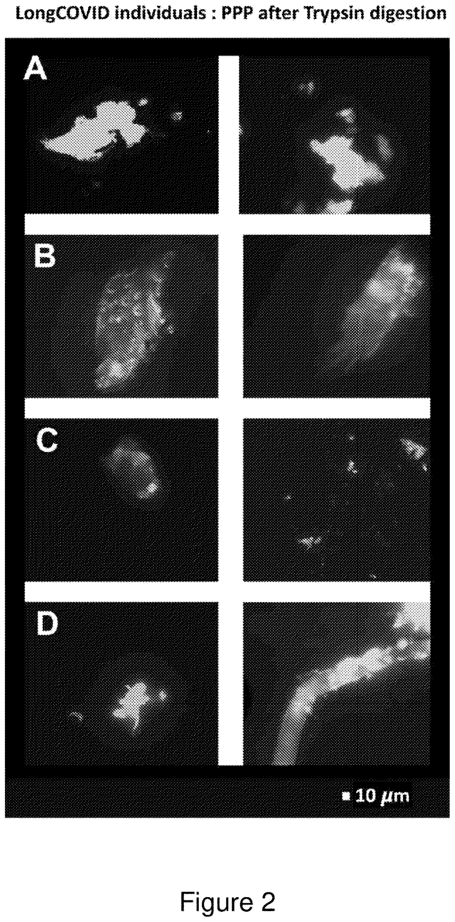

**Figures 2** **A to D** show representative micrographs of plasma smears, after fibrinolysis treatment with trypsin fibrinolysis from four of the six patients who suffered from acute COVID-19, but who recovered. They are now suffering from LongCOVID two months after their recovery from acute COVID-19.

Using fluorescence microscopy to view the plasma samples treated according to the method described above, very little anomalous (amyloid) fluorescent signal was visible in the plasma of type 2 diabetes and healthy individuals. In contrast, using the same method, the COVID-19, as well as LongCOVID plasma samples, contained large insoluble fluorescent deposits. This suggests that trypsin does not fully digest plasma samples from COVID-19 or LongCOVID patients.

### EXAMPLE 2

### Fluorescent microscopy of plasma (before trypsin digestion)

### Patient Samples

All six LongCOVID patients showed anomalous (amyloid) clotlets before trypsin digestion.

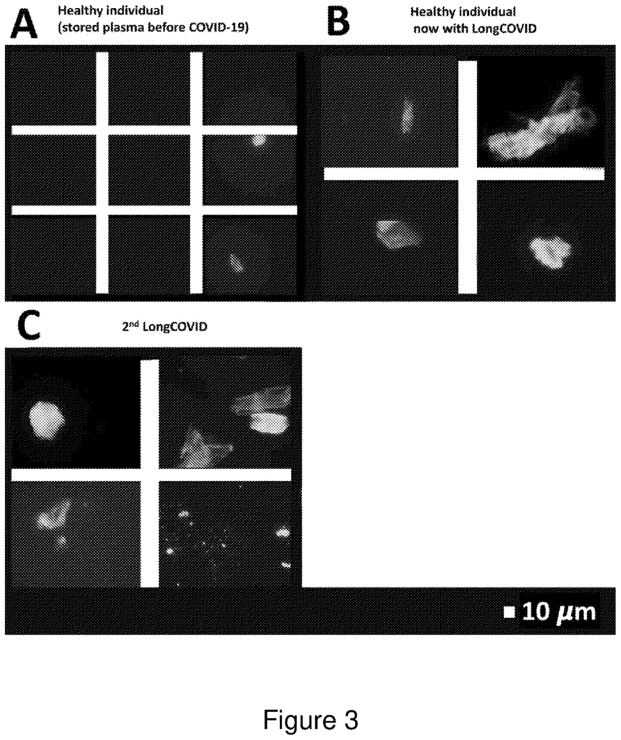

Figure 3(A) and (B) show plasma samples of the same patent exposed to ThT. Figure 3(A) shows the stored plasma sample of patient taken in 2018, before acute COVID-19 infection. Figure 3(B) shows the plasma sample of patient, two months after acute COVID-19 recovery - this patient is now suffering from LongCOVID. Figure 3(C) shows a plasma sample of 2^{nd} patient, two months after recovery from acute COVID-19, now suffering from LongCOVID.

The LongCOVID patients were confirmed to be COVID-positive, when diagnosed with acute COVID-19, by PRC testing at a pathology laboratory.

Further, for all LongCOVID plasma samples, it was shown that the anomalous clotlets are present before fibrinolytic digestion with trypsin. This suggests that the method can be used to detect LongCOVID both with or without the addition of a fibrinolytic enzyme.

### Fluorescent microscopy without fibrinolytic treatment

Following the preparation of platelet poor plasma from whole blood, 0.5 µL Thioflavin T (ThT), at a 5 miliMolar final concentration, was added to the 10 µL plasma and left for 30 minutes. Thereafter, 3 µL of the sample was placed onto a microscope slide, left for 30 seconds, and a coverslip was placed onto the sample. The samples were viewed using a Zeiss Axio Observer 7 fluorescent microscope with a Plan-Apochromat 63x/1.4 Oil DIC M27 objective (Carl Zeiss Microscopy, Munich, Germany). The excitation wavelength for ThT was set at 450nm to 488nm and the emission at 499nm to 529nm.

Figure 3 A is a micrograph of a comparative sample from one of the patients before COVID-19 (plasma was collected in 2018, prior to the emergence of SARS-CoV-2) and Figure 2 B is a micrograph of a sample collected from the same patient after recovery from acute COVID-19 but during subsistence of enduring symptoms indicative of LongCOVID. Figure 3 C shows representative micrographs of plasma smears from a second patient who suffered from acute COVID-19, but who recovered. Persistent anomalous (amyloid) clotlets are still present in their plasma even before fibrinolytic treatment.

## Claims

1. An in vitro method of diagnosing post-acute COVID-19 syndrome in a subject comprising:
(i) providing a sample that has been obtained from the subject;
(ii) treating the sample with a fluorescent dye or fluorescent probe; and
(iii) using a fluorescence or microscopy detection method to detect the fluorescent dye or fluorescent probe thereby detecting for a presence of persistent anomalous amyloid clotlets having a diameter of 1 to 1000 µm in the sample,
wherein the presence of persistent anomalous amyloid clotlets in the sample is indicative of post-acute COVID-19 syndrome in the subject.

2. The method of claim 1, wherein the sample is a blood sample.

3. The method of claim 2, further comprising a step of preparing platelet poor plasma (PPP) from the blood sample.

4. The method of any one of claims 1 to 3, wherein the method comprises treating the sample with a fibrinolytic enzyme prior to the step of using the fluorescence or microscopy detection method to detect for the presence of persistent anomalous amyloid clotlets in the sample.

5. The method of claim 4, wherein the fibrinolytic enzyme is selected from the group consisting of trypsin, protein kinases, plasmin, anistreplase, desmoteplase, streptokinase, urokinase, nattokinase, lumbrokinase, serrapeptase, papain, DNase, bromelain, and honokiol.

6. The method of any one of claims 1 to 5, wherein the fluorescence or microscopy detection method is fluorescence microscopy, confocal microscopy, fluorescence spectrofluorometry, or flow cytometry.

## Patentansprüche

1. In-vitro-Verfahren zur Diagnose von postakutem COVID-19-Syndrom bei einem Subjekt, das Folgendes umfasst:
(i) Bereitstellen einer Probe, die von dem Subjekt erhalten wurde;
(ii) Behandeln der Probe mit einem fluoreszierenden Farbstoff oder einer fluoreszierenden Sonde; und
(iii) Verwenden eines Fluoreszenz- oder Mikroskopiedetektionsverfahrens, um den fluoreszierenden Farbstoff oder die fluoreszierende Sonde zu detektieren, wodurch eine Gegenwart von persistenten anomalen amyloiden Gerinnseln, die einen Durchmesser von 1 bis 1000 µm aufweisen, in der Probe detektiert wird,
wobei die Gegenwart von persistenten anomalen amyloiden Gerinnseln in der Probe ein postakutes COVID-19-Syndrom bei dem Subjekt anzeigt.

2. Verfahren nach Anspruch 1, wobei die Probe eine Blutprobe ist.

3. Verfahren nach Anspruch 2, das weiter einen Schritt der Herstellung von plättchenarmem Plasma (PPP) aus der Blutprobe umfasst.

4. Verfahren nach einem der Ansprüche 1 bis 3, wobei das Verfahren das Behandeln der Probe mit einem fibrinolytischen Enzym vor dem Schritt der Verwendung des Fluoreszenz- oder Mikroskopiedetektionsverfahrens umfasst, um die Gegenwart von persistenten anomalen amyloiden Gerinnseln in der Probe zu detektieren.

5. Verfahren nach Anspruch 4, wobei das fibrinolytische Enzym aus der Gruppe ausgewählt ist, die aus Folgenden besteht: Trypsin, Proteinkinasen, Plasmin, Anistreplase, Desmoteplase, Streptokinase, Urokinase, Nattokinase, Lumbrokinase, Serrapeptase, Papain, DNase, Bromelain und Honokiol.

6. Verfahren nach einem der Ansprüche 1 bis 5, wobei das Fluoreszenz- oder Mikroskopiedetektionsverfahren Fluoreszenzmikroskopie, konfokale Mikroskopie, Fluoreszenz-Spektrofluorometrie oder Durchflusszytometrie ist.

## Revendications

1. Méthode *in vitro* destinée au diagnostic d'un syndrome de post-COVID-19 aigüe chez un sujet, comprenant :
(i) la mise à disposition d'un échantillon qui a été obtenu à partir du sujet :
(ii) le traitement de l'échantillon par un colorant fluorescent ou une sonde fluorescente ; et
(iii) l'utilisation d'une méthode de détection par fluorescence ou microscopie afin de détecter le colorant fluorescent ou la sonde fluorescente, et de détecter ainsi la présence dans l'échantillon de micro-caillots amyloïdes anormaux persistants présentant un diamètre allant de 1 à 1000 µm,
la présence de micro-caillots amyloïdes anormaux persistants dans l'échantillon indiquant un syndrome de post-COVID-19 aigüe chez le sujet.

2. Méthode selon la revendication 1, dans laquelle l'échantillon est un échantillon de sang.

3. Méthode selon la revendication 2, comprenant en outre une étape de préparation de plasma pauvre en plaquettes (PPP) à partir de l'échantillon de sang.

4. Méthode selon l'une quelconque des revendications 1 à 3, la méthode comprenant le traitement de l'échantillon par une enzyme fibrinolytique avant l'étape d'utilisation de la méthode de détection par fluorescence ou microscopie afin de détecter la présence de micro-caillots amyloïdes anormaux persistants dans l'échantillon.

5. Méthode selon la revendication 4, dans laquelle l'enzyme fibrinolytique est choisie dans le groupe constitué par la trypsine, les protéines kinases, la plasmine, l'anistréplase, la desmotéplase, la streptokinase, l'urokinase, la nattokinase, la lumbrokinase, la serrapeptase, la papaïne, la DNase, la bromélaïne et l'honokiol.

6. Méthode selon l'une quelconque des revendications 1 à 5, dans laquelle la méthode de détection par fluorescence ou microscopie est la microscopie à fluorescence, la microscopie confocale, la spectroscopie de fluorescence ou la cytométrie en flux.
